(19) Europäisches Patentamt
European Patent Office
Office européen des brevets

(11) **EP 3 120 798 B1**

(12) **FASCICULE DE BREVET EUROPEEN**

(45) Date de publication et mention
de la délivrance du brevet:
**03.10.2018 Bulletin 2018/40**

(51) Int Cl.:
***A61B 34/20*** *(2016.01)* ***A61B 90/00*** *(2016.01)*

(21) Numéro de dépôt: **16179117.3**

(22) Date de dépôt: **12.07.2016**

(54) **SYSTÈME ÉLECTROMAGNÉTIQUE DE SUIVI DE POSITION**

ELEKTROMAGNETISCHES SYSTEM ZUM NACHVERFOLGEN EINER POSITION

ELECTROMAGNETIC POSITION TRACKING SYSTEM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.07.2015 FR 1556827**

(43) Date de publication de la demande:
**25.01.2017 Bulletin 2017/04**

(73) Titulaires:
• **COMMISSARIAT À L'ÉNERGIE ATOMIQUE ET AUX
ÉNERGIES ALTERNATIVES
75015 Paris (FR)**
• **MinMaxMedical
38700 La Tronche (FR)**

(72) Inventeurs:
• **BERTRAND, François
38180 SEYSSINS (FR)**
• **JOSSELIN, Vincent
38000 GRENOBLE (FR)**
• **HUGUEL, Loïc
38700 LA TRONCHE (FR)**
• **ROUSSEAU, Sandra
38700 LA TRONCHE (FR)**

(74) Mandataire: **Cabinet Beaumont
4, Place Robert Schuman
B.P. 1529
38025 Grenoble Cedex 1 (FR)**

(56) Documents cités:
**US-A1- 2003 184 285**

EP 3 120 798 B1

**Description**

Domaine

La présente demande concerne le domaine des systèmes électromagnétiques de suivi de position ou systèmes électromagnétiques de localisation. Elle vise plus particulièrement des systèmes électromagnétiques de suivi de position adaptés au domaine médical, par exemple pour la localisation d'un ou plusieurs outils utilisés par un chirurgien au cours d'une intervention médicale.

Exposé de l'art antérieur

On s'intéresse ici plus particulièrement à des systèmes de suivi de position du type comportant :

un dispositif d'émission d'un champ électromagnétique comportant une ou plusieurs bobines d'émission ; et
un dispositif de réception de ce champ comportant une ou plusieurs bobines de réception.

Dans un tel système, l'analyse conjointe des champs émis par les bobines d'émission et des champs reçus par les bobines de réception permet de déterminer la position et/ou l'orientation du dispositif de réception par rapport au dispositif d'émission.

Un exemple d'un tel système est par exemple décrit dans la demande de brevet US2005/0165297.

Il existe un besoin pour un système électromagnétique de suivi de position présentant des performances améliorées par rapport aux systèmes connus, notamment en matière de précision de localisation.

Résumé

Ainsi, un mode de réalisation prévoit un système électromagnétique de suivi de position, comprenant : un dispositif d'émission d'un champ électromagnétique comportant au moins une bobine d'émission et, connecté à la bobine d'émission, un premier générateur d'un signal électrique d'excitation de la bobine d'émission ; un dispositif de réception du champ électromagnétique émis par le dispositif d'émission, comportant au moins une bobine de réception et, connecté à la bobine de réception, un circuit de lecture d'un signal électrique induit dans la bobine de réception ; et un système de mesure d'au moins un paramètre du dispositif de réception, comprenant, directement connecté à la bobine de réception par une piste ou un fil conducteur, un deuxième générateur d'un signal électrique d'excitation de la bobine de réception.

Selon un mode de réalisation, le paramètre est un paramètre du groupe comprenant la résistance série et l'inductance propre de la bobine de réception.

Selon un mode de réalisation, le dispositif de réception comprend au moins deux bobines de réception, et le paramètre est un paramètre du groupe comprenant la résistance série de chaque bobine de réception, l'inductance propre de chaque bobine de réception, et le coefficient d'inductance mutuelle entre deux bobines de réception.

Selon un mode de réalisation, le paramètre comprend la résistance série de chaque bobine de réception, et le système de mesure est adapté, pour mesurer la résistance série, à appliquer sur une extrémité de la bobine de réception une tension continue ou une tension sinusoïdale de fréquence au moins 20 fois plus faible que la fréquence de coupure de la bobine.

Selon un mode de réalisation, le paramètre comprend l'inductance propre de chaque bobine de réception, et le système de mesure est adapté, pour mesurer l'inductance propre, à appliquer sur une extrémité de la bobine de réception une tension sinusoïdale de fréquence supérieure à la fréquence de coupure de la bobine.

Selon un mode de réalisation, le paramètre comprend le coefficient d'inductance mutuelle entre deux bobines de réception, et le système de mesure est adapté, pour mesurer le coefficient, à appliquer sur une extrémité de l'une des deux bobines de réception une tension sinusoïdale de fréquence supérieure à la fréquence de coupure de la bobine.

Selon un mode de réalisation, le circuit de lecture comprend, associé à chaque bobine de réception, un étage d'amplification de lecture comportant un amplificateur opérationnel dont l'entrée inverseuse est reliée à la sortie par l'intermédiaire d'une résistance de contre-réaction et dont l'entrée non inverseuse est reliée à un noeud d'application d'un potentiel de référence, une première extrémité de la bobine de réception étant connectée à l'entrée inverseuse de l'amplificateur opérationnel.

Selon un mode de réalisation, le deuxième générateur comprend, associé à chaque bobine de réception, une source de tension commandable connectée entre la deuxième extrémité de la bobine de réception et le noeud d'application d'un potentiel de référence.

Selon un mode de réalisation, le deuxième générateur comprend un synthétiseur numérique de fréquences.

Selon un mode de réalisation, la bobine d'émission comprend trois bobines d'émission orientées selon des axes distincts, et la bobine de réception comprend trois bobines de réception orientées selon des axes distincts.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente schématiquement, sous forme de blocs, un exemple d'un système électromagnétique de suivi de position ;
la figure 2 représente plus en détail une partie du dispositif de réception du système électromagnétique de suivi de position de la figure 1 ;
la figure 3 représente schématiquement, sous forme de blocs, un exemple d'un mode de réalisation d'un système électromagnétique de suivi de position ; et
la figure 4 représente plus en détail une partie du système électromagnétique de suivi de position de la figure 3.

Description détaillée

De mêmes éléments ont été désignés par de mêmes références aux différentes figures. Par souci de clarté, seuls les éléments qui sont utiles à la compréhension des modes de réalisation décrits ont été représentés et sont détaillés. En particulier, ne sont pas détaillés les algorithmes de calcul permettant, à partir des valeurs des champs émis par les bobines d'émission et reçus par les bobines de réception, de déterminer la position et/ou l'orientation du dispositif de réception par rapport au dispositif d'émission. En effet, les modes de réalisation décrits sont compatibles avec les algorithmes usuels de détermination des informations de position et/ou d'orientation du dispositif de réception à partir des valeurs de champ mesurées. Sauf précision contraire, les expressions "sensiblement" et "de l'ordre de" signifient à 10 % près, de préférence à 5 % près. Par ailleurs, dans la présente description, on utilisera le terme "connecté" pour désigner une liaison électrique directe, sans composant électronique intermédiaire, par exemple au moyen d'une ou plusieurs pistes conductrices ou fils conducteurs, et le terme "couplé" ou le terme "relié", pour désigner soit une liaison électrique directe (signifiant alors "connecté") soit une liaison via un ou plusieurs composants intermédiaires (résistance, condensateur, etc.).

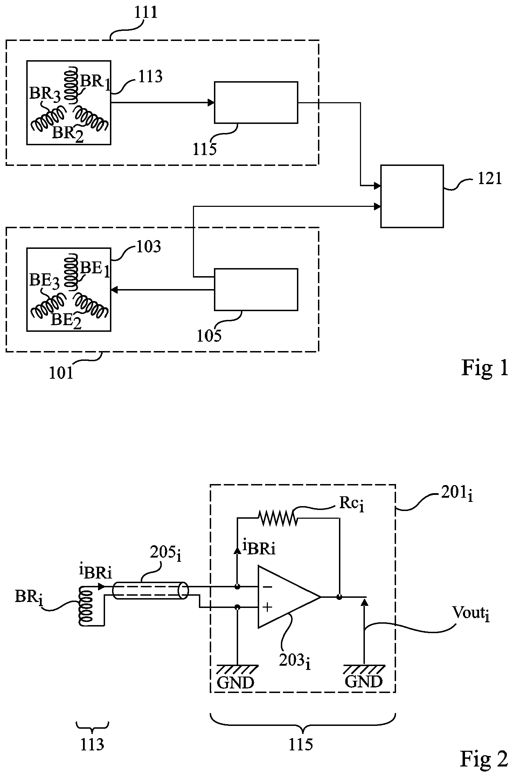

La figure 1 représente schématiquement, sous forme de blocs, un exemple d'un système électromagnétique de suivi de position.

Le système de la figure 1 comprend un dispositif 101 d'émission d'un champ électromagnétique comprenant un ensemble 103 d'une ou plusieurs bobines d'émission. On considère ici, à titre d'exemple, le cas d'un système dans lequel l'ensemble 103 comprend trois bobines d'émission $BE_1$, $BE_2$ et $BE_3$ orientées selon trois axes distincts, par exemple selon les trois axes d'un repère orthogonal. Le dispositif d'émission 101 comprend en outre un dispositif 105 de génération de signaux électriques (tensions ou courants) alternatifs d'excitation des bobines de l'ensemble 103, par exemple des signaux sinusoïdaux. Le générateur 105 est connecté à au moins une borne de chacune des bobines $BE_1$, $BE_2$ et $BE_3$.

Le système de la figure 1 comprend en outre un dispositif 111 de réception du champ électromagnétique émis par le dispositif d'émission 101. Le dispositif de réception 111 comprend un ensemble 113 d'une ou plusieurs bobines de réception. On considère ici, à titre d'exemple, le cas d'un système dans lequel l'ensemble 113 comprend trois bobines de réception $BR_1$, $BR_2$ et $BR_3$ orientées selon trois axes distincts, par exemple selon les trois axes d'un repère orthogonal. Le dispositif de réception 111 comprend en outre un dispositif 115 de lecture des signaux électriques (tensions ou courants) induits dans les bobines de l'ensemble 113 sous l'effet des champs alternatifs émis par les bobines d'émission.

Le système de la figure 1 comprend en outre un dispositif de traitement 121, comprenant par exemple un microprocesseur, relié d'une part au générateur 105 du dispositif d'émission 101 et d'autre part au dispositif de lecture 115 du dispositif de réception 111. Les liaisons entre le générateur 105 et le dispositif de traitement 121 et entre le dispositif de lecture 115 et le dispositif de traitement 121 peuvent être des liaisons filaires ou des liaisons sans fil. Le générateur 105 est adapté à transmettre au dispositif de traitement 121 des signaux représentatifs des signaux d'excitation qu'il applique aux bornes des bobines d'émission $BE_1$, $BE_2$ et $BE_3$. Le dispositif de lecture 115 est adapté à transmettre au dispositif de traitement 121 des signaux représentatifs des signaux lus aux bornes des bobines de réception $BR_1$, $BR_2$ et $BR_3$. Les signaux transmis par les dispositifs 105 et 115 au dispositif de traitement 121 sont par exemple des signaux numériques. A titre d'exemple, le générateur 105 comprend un circuit (non représenté) d'échantillonnage et de numérisation des signaux analogiques d'excitation appliqués aux bornes des bobines d'émission $BE_1$, $BE_2$ et $BE_3$, et le dispositif de lecture 115 comprend un circuit (non représenté) d'échantillonnage et de numérisation des signaux analogiques lus aux bornes des bobines de réception $BR_1$, $BR_2$ et $BR_3$.

Le fonctionnement du système de localisation de la figure 1 est le suivant.

L'ensemble 103 de bobines d'émission du dispositif d'émission 101 définit un référentiel pour la localisation de l'ensemble 113 de bobines de réception du dispositif de réception 111. L'ensemble 113 de bobines de réception peut être fixé sur un objet à localiser. A titre d'exemple, dans le cas d'une application à la localisation d'un outil chirurgical, l'ensemble 103 de bobines d'émission peut être fixé sur le corps d'un patient ou sur une structure externe au corps du patient servant de référentiel. L'ensemble 113 de bobines de réception peut être fixé sur un outil chirurgical que l'on souhaite pouvoir localiser pendant l'intervention.

Lors d'une phase de suivi de la position de l'objet à localiser, les bobines d'émission $BE_1$, $BE_2$ et $BE_3$ du dispositif d'émission 101, commandées par le générateur 105, émettent chacune un champ électromagnétique alternatif. A titre d'exemple, le générateur 105 est configuré pour appliquer simultanément des signaux d'excitation de fréquences distinctes aux différentes bobines de l'ensemble 103, auquel cas les bobines de l'ensemble 103 émettent simultanément des champs électromagnétiques alternatifs de fréquences distinctes. Ceci permet, au niveau du dispositif de réception, de pouvoir discriminer spectralement les champs provenant des différentes bobines d'émission. A titre de variante, le générateur est configuré pour appliquer séquentiellement aux différentes bobines de l'ensemble 103 des signaux d'excitation de même fréquence, de façon qu'à un instant donné, seule l'une des bobines de l'ensemble 103 émette un champ électromagnétique. Ceci permet, au niveau du dispositif de réception, de pouvoir discriminer temporellement les champs provenant des différentes bobines d'émission. La ou les fréquences d'émission du dispositif 101 sont par exemple comprises dans la plage allant de 1 à 50 kHz. Des signaux (par exemple numériques) représentatifs des signaux d'excitation appliqués par le générateur 105 aux bobines $BE_1$, $BE_2$ et $BE_3$, sont transmis à l'unité de traitement 121.

Au niveau du dispositif de réception 111, des signaux électriques sont induits dans les bobines de réception $BR_1$, BR2 et $BR_3$ sous l'effet des champs électromagnétiques émis par les bobines d'émission $BE_1$, $BE_2$ et $BE_3$. Ces signaux sont mesurés par le dispositif de lecture 115 et transmis (par exemple sous forme numérique) à l'unité de traitement 121.

Le dispositif de traitement 121 comprend une unité de démodulation synchrone et de filtrage (non visible sur la figure) adaptée, à partir des signaux transmis par le générateur 105 et le dispositif de lecture 115, à déterminer, pour chacune des bobines de réception de l'ensemble 113, l'intensité du champ capté par la bobine en provenance de chacune des bobines d'émission de l'ensemble 103. A partir de l'intensité des champs captés par les bobines de réception, le dispositif de traitement 121 détermine la position et/ou l'orientation de l'ensemble de bobines de réception 113 par rapport à l'ensemble de bobines d'émission 103. Plus particulièrement, dans l'exemple de la figure 1, l'unité de démodulation synchrone et de filtrage détermine neuf valeurs de champ correspondant respectivement au champ reçu par la bobine $BR_1$ en provenance de la bobine $BE_1$, au champ reçu par la bobine $BR_1$ en provenance de la bobine $BE_2$, au champ reçu par la bobine $BR_1$ en provenance de la bobine $BE_3$, au champ reçu par la bobine $BR_2$ en provenance de la bobine $BE_1$, au champ reçu par la bobine $BR_2$ en provenance de la bobine $BE_2$, au champ reçu par la bobine $BR_2$ en provenance de la bobine $BE_3$, au champ reçu par la bobine $BR_3$ en provenance de la bobine $BE_1$, au champ reçu par la bobine $BR_3$ en provenance de la bobine $BE_2$, et au champ reçu par la bobine $BR_3$ en provenance de la bobine $BE_3$. A partir de ces neuf valeurs de champ, l'unité de traitement 121 calcule les coordonnées spatiales de position de l'ensemble de bobines de réception 113 selon les trois axes du repère défini par les bobines d'émission, et trois valeurs angulaires définissant l'orientation de l'ensemble de bobines de réception 113 par rapport aux axes du repère défini par les bobines d'émission (on parle d'un système de localisation à six degrés de liberté).

La précision d'un système de localisation du type décrit en relation avec la figure 1 dépend notamment de la précision de la mesure des champs captés par les bobines du dispositif de réception. Ainsi, pour obtenir une bonne précision de localisation, il convient de connaître avec précision la fonction de transfert de conversion en valeurs de champs des signaux électriques (tensions ou courants) mesurés aux bornes des bobines du dispositif de réception 111.

On s'intéresse ici plus particulièrement au cas où le dispositif de lecture 115 du dispositif de réception 111 comprend un dispositif d'amplification de type transimpédance. Un exemple d'un tel dispositif d'amplification est illustré en figure 2.

La figure 2 représente plus en détail une partie du dispositif de réception 111 d'un système électromagnétique de suivi de position du type décrit en relation avec la figure 1.

Dans l'exemple de la figure 2, le dispositif de lecture 115 comprend, associé à chacune des bobines de réception $BR_i$, avec i entier allant de 1 à 3, un dispositif d'amplification $201_i$ de type transimpédance, c'est-à-dire comportant un amplificateur opérationnel $203_i$ dont l'entrée inverseuse (-) est reliée à la sortie par une résistance de contre-réaction $Rc_i$ et dont l'entrée non-inverseuse (+) est reliée à un noeud d'application d'un potentiel de référence GND, par exemple la masse. Par souci de simplification, seule une bobine $BR_i$ et un dispositif d'amplification $201_i$ associé à cette bobine ont été représentés sur la figure 2.

La bobine $BR_i$ a ses deux extrémités connectées, par exemple par l'intermédiaire d'un câble coaxial $205_i$, respectivement à l'entrée inverseuse (-) et à l'entrée non-inverseuse (+) de l'amplificateur opérationnel $203_i$.

Dans un tel montage, la bobine $BR_i$ est mise en court-circuit du fait de la masse virtuelle présente sur l'entrée inverseuse (-) de l'amplificateur opérationnel $203_i$. La bobine $BR_i$ est traversée par un courant $i_{BRi}$ proportionnel à l'intensité du champ magnétique reçu par la bobine.

En fonctionnement, l'amplificateur opérationnel $203_i$ fournit sur son noeud de sortie une tension $Vout_i$ (référencée par rapport au noeud GND) image amplifiée du courant $i_{BRi}$.

Le dispositif de lecture 115 peut en outre comprendre, associé à chacune des bobines de réception $BR_i$, un circuit d'échantillonnage et de numérisation (non représenté) adapté à échantillonner et numériser la tension $Vout_i$, la sortie numérique de ce circuit étant reliée au dispositif de traitement 121.

La fonction de transfert ou gain de conversion champ magnétique/tension d'un tel montage peut s'exprimer de la manière suivante :

$$K_{BRi}(j\omega) = \frac{V_{outi}}{B} = -\frac{M_{BRi}}{L_{BRi}} Rc_i \frac{\frac{L_{BRi}}{Rs_{BRi}}j\omega}{1 + \frac{L_{BRi}}{Rs_{BRi}}j\omega}$$

où B désigne l'intensité du champ magnétique reçu par la bobine $BR_i$, j désigne l'unité imaginaire, $\omega$ désigne la pulsation du champ B ($\omega=2\pi f$, f étant la fréquence du champ B), $M_{BRi}$ désigne le moment magnétique par unité de courant de la bobine $BR_i$, en $A.m^2/A$, $L_{BRi}$ désigne l'inductance de la bobine $BR_i$, $Rs_{BRi}$ désigne la résistance série du circuit connecté entre l'entrée inverseuse (-) et l'entrée non inverseuse (+) de l'amplificateur opérationnel $203_i$, comprenant principalement, dans cet exemple, la résistance de la bobine $BR_i$ et la résistance du câble $205_i$, et $K_{BRi}(j\omega)$ désigne le coefficient de proportionnalité champ/tension à la pulsation $\omega$.

La réponse en fréquence du gain de conversion $K_{BRi}$ est une réponse de type passe-haut, de gain égal à $(M_{BRi}/L_{BRi})*Rc_i$, et de fréquence de coupure égale à $Rs_{BRi}/(2\pi L_{BRi})$.

La connaissance précise de la fonction de transfert champ magnétique/tension d'un tel montage nécessite de connaître précisément les paramètres $L_{BRi}$ et $Rs_{BRi}$ du montage.

La connaissance précise de la valeur de la résistance $Rs_{BRi}$ pose tout particulièrement problème car cette valeur est susceptible de varier dans des proportions relativement importantes sous l'effet des variations de températures auxquelles peuvent être soumises les bobines de réception $BR_i$.

La valeur de l'inductance $L_{BRi}$, bien que moins dépendante des variations de température, reste malgré tout susceptible de subir des dérives (dépendant notamment des matériaux utilisés, comme par exemple l'utilisation ou non d'un noyau ferromagnétique). La connaissance précise de cette valeur peut par ailleurs être utile dans une phase de calibration du système électromagnétique pour contraindre certains coefficients utilisés par l'algorithme de calcul de détermination de la position et/ou de l'orientation.

Par ailleurs, dans le cas d'un dispositif de réception à plusieurs bobines du type représenté en figure 1, il se produit un phénomène de couplage parasite des bobines de réception $BR_i$ les unes avec les autres. Plus particulièrement, chaque bobine réémet un champ magnétique parasite proportionnel au courant induit qui la traverse, ce champ parasite étant lui-même capté par les autres bobines de réception. Ainsi, par exemple, dans la configuration à trois bobines de réception de la figure 1, le champ B capté par la bobine de réception $BR_3$ comprendra non seulement le champ utile que l'on souhaite mesurer, à savoir le champ émis par les bobines $BE_1$, $BE_2$ et $BE_3$ du dispositif d'émission 101, mais aussi un champ parasite réémis par la bobine de réception $BR_1$, et un champ parasite réémis par la bobine de réception $BR_2$. Pour déterminer le champ utile capté par chacune des bobines $BR_i$, il convient de connaître les champs parasites émis par les autres bobines de réception de l'ensemble 113. Pour cela, il convient de connaître avec précision les coefficients d'inductance mutuelle entre les différentes bobines de réception. En particulier, dans le cas d'un montage transimpédance du type décrit en relation avec la figure 2, le champ parasite reçu par une bobine de réception $BR_i$ en provenance d'une autre bobine de réception $BR_{i'}$ (avec i' entier allant de 1 à 3 différent de i) est égal à

$$\frac{Ml_{i,i'}}{M_{BRi}} i_{BRi'} = \frac{Ml_{i,i'}}{M_{BRi}} \frac{V_{outi'}}{Rc_{i'}}$$

où $Ml_{i,i'}$ désigne le coefficient d'inductance mutuelle entre les bobines $BR_i$ et $BR_{i'}$.

Les valeurs des coefficients d'inductance mutuelle sont peu dépendantes des variations de température, et fluctuent peu sur le court terme. Toutefois, de même que pour les valeurs d'inductance $L_{BRi}$, la connaissance des coefficients d'inductance mutuelle peut être utile dans la phase de calibration du système électromagnétique pour contraindre certains coefficients utilisés par l'algorithme de calcul de détermination de la position et/ou de l'orientation.

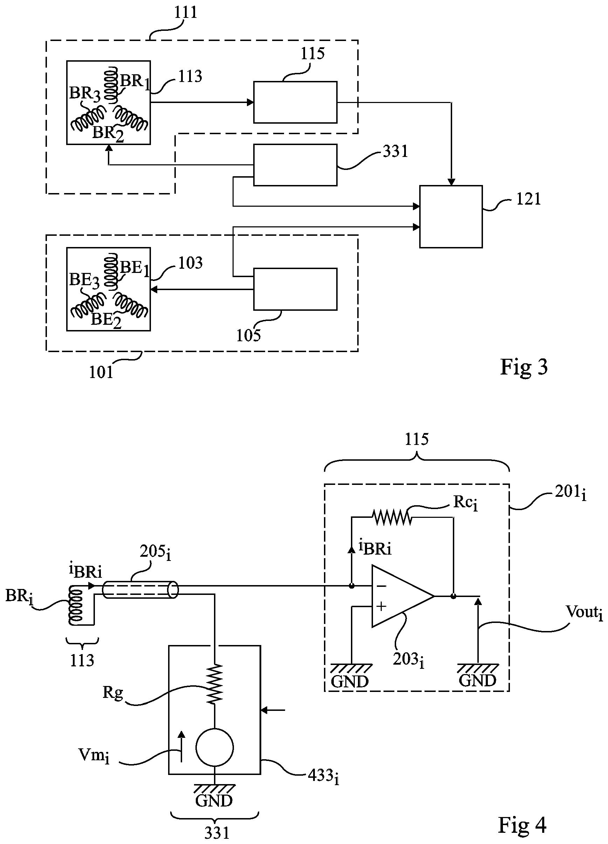

La figure 3 représente schématiquement, sous forme de blocs, un exemple d'un mode de réalisation d'un système électromagnétique de suivi de position.

Le système de la figure 3 comprend des éléments communs avec le système de la figure 1. Ces éléments ne seront pas détaillés à nouveau. Dans la description qui suit, seules les différences entre le système de la figure 3 et le système de la figure 1 seront détaillées.

Le système de la figure 3 diffère du système de la figure 1 en ce qu'il comprend, outre le dispositif d'émission 101, le dispositif de réception 111 et l'unité de traitement 121, un système de mesure de paramètres du dispositif de réception 111, ce système de mesure permettant de déterminer in-situ, c'est-à-dire pendant une phase d'utilisation du système de localisation pour suivre la position d'un objet, les valeurs des paramètres $Rs_{BRi}$ et/ou $L_{BRi}$ et/ou $Ml_{i,i'}$ du montage formé par les bobines de réception $BR_i$ et les étages d'amplification de lecture $201_i$ associés aux bobines de réception.

Le système de mesure de paramètres comprend un dispositif commandable 331 de génération de signaux électriques (tensions ou courants) d'excitation des bobines de réception de l'ensemble 113. Le générateur 331 est connecté à au moins une borne de chacune des bobines de réception $BR_1$, $BR_2$ et $BR_3$.

Le générateur 331 est relié au dispositif de traitement 121 par une liaison filaire ou sans fil. Le générateur 331 est adapté à transmettre au dispositif de traitement 121 des signaux (par exemple numériques) représentatifs des signaux d'excitation qu'il applique aux bornes des bobines de réception $BR_1$, $BR_2$ et $BR_3$.

La figure 4 représente plus en détail une partie du système de localisation de la figure 3.

Dans l'exemple de la figure 4, le dispositif de lecture 115 comprend, comme dans l'exemple de la figure 2, associé à chacune des bobines de réception $BR_i$, un dispositif d'amplification $201_i$ de type transimpédance, comportant un amplificateur opérationnel $203_i$ dont l'entrée inverseuse (-) est reliée à la sortie par une résistance de contre-réaction $Rc_i$ et dont l'entrée non-inverseuse (+) est reliée à un noeud d'application d'un potentiel de référence GND, par exemple la masse.

Dans l'exemple de la figure 4, le générateur 331 comprend, associé à chacune des bobines de réception $BR_i$, une source de tension commandable $433_i$ connectée à une extrémité de la bobine $BR_i$ et adaptée à appliquer une tension $Vm_i$, référencée par rapport au noeud GND, sur cette extrémité de la bobine $BR_i$.

L'autre extrémité de la bobine $BR_i$ est connectée à l'entrée inverseuse (-) de l'amplificateur opérationnel $203_i$.

Par souci de simplification, seuls une bobine $BR_i$, un dispositif d'amplification $201_i$ associé à cette bobine, et une source de tension commandable 433i associée à cette bobine ont été représentés sur la figure 4.

A titre d'exemple, un câble $205_i$, par exemple un câble coaxial, relie les deux extrémités de la bobine $BR_i$ respectivement à l'entrée inverseuse (-) de l'amplificateur opérationnel $203_i$ et à la sortie de la source de tension commandable $433_i$.

Dans ce montage, la bobine $BR_i$ n'est pas connectée directement à l'entrée non inverseuse (+) de l'amplificateur opérationnel $203_i$, mais est reliée à l'entrée non inverseuse (+) par l'intermédiaire de la source de tension commandable $433_i$ et du noeud de référence GND.

Le fonctionnement du système électromagnétique de localisation des figures 3 et 4 est identique ou similaire à celui des figures 1 et 2 en ce qui concerne les opérations de suivi de position à proprement parler, à la différence près que, dans le système des figures 3 et 4, la résistance série $Rs_{BRi}$ associée à la bobine de réception $BR_i$, c'est-à-dire la résistance série du circuit connecté entre l'entrée inverseuse (-) et l'entrée non inverseuse (+) de l'amplificateur opérationnel $203_i$, comprend non seulement la résistance de la bobine $BR_i$ et la résistance du câble $205_i$, mais comprend en outre la résistance série $R_g$ de la source de tension commandable $433_i$.

Le fonctionnement du système de mesure de paramètres du dispositif de réception 101 du système électromagnétique de localisation des figures 3 et 4 va maintenant être décrit dans les trois cas suivants : A) pour mesurer la résistance série $Rs_{BRi}$ associée à une bobine de réception $BR_i$, B) pour mesurer l'inductance $L_{BRi}$ d'une bobine de réception $BR_i$, et C) pour mesurer le coefficient d'inductance mutuelle $Ml_{i,i'}$ entre deux bobines de réception $BR_i$ et $BR_{i'}$.

A) mesure de la résistance série $Rs_{BRi}$

Pour la mesure de la résistance série $Rs_{BRi}$ associée à une bobine $BR_i$, le générateur 331 commande l'application, via la source de tension commandable $433_i$, d'une tension $Vm_i$ continue ou sinusoïdale sur la borne de la bobine $BR_i$ connectée à la source de tension $433_i$.

Dans le cas d'une tension $Vm_i$ sinusoïdale, cette dernière peut s'exprimer comme suit :

$$Vm_i(t) = V_{ref} \cos(2\pi f_i t)$$

où $V_{ref}$ désigne l'amplitude de la tension sinusoïdale et $f_i$ sa fréquence.

La tension $Vm_i$ est amplifiée par l'étage d'amplification transimpédance $201_i$, l'image amplifiée de cette tension formant une composante de la tension de sortie $V_{outi}$ de l'étage d'amplification $201_i$. La fréquence $f_i$ de la tension $Vm_i$ est de préférence choisie très inférieure à la fréquence de coupure de la bobine (égale à $RS_{BRi}/2\pi L_{BRi}$), de sorte que l'impédance de l'inductance de la bobine $BR_i$ puisse être négligée devant la résistance série à mesurer. A titre d'exemple, la fréquence $f_i$ est choisie au moins 20 fois plus faible que la fréquence de coupure de la bobine. A titre d'exemple, la

fréquence $f_i$ peut être comprise dans la plage allant de 1 à 20 Hz pour une fréquence de coupure de la bobine sensiblement égale à 1 kHz. L'amplitude $V_{ref}$ de la tension sinusoïdale est choisie de manière à ne pas saturer l'amplificateur opérationnel $203_i$, c'est-à-dire de façon que la tension de sortie $V_{outi}$ de l'étage d'amplification reste inférieure à la tension de sortie de saturation de l'amplificateur 203i.

Le dispositif de traitement 121 comprend une unité de détection synchrone (non visible sur la figure) adaptée à extraire la valeur crête $V_{outifi}$ de la composante de fréquence $f_i$ du signal $V_{outi}$. A partir de cette valeur crête $V_{outifi}$, le dispositif de traitement 121 peut déterminer la valeur de la résistance série $Rs_{BRi}$ par la formule suivante :

$$Rs_{BRi} = \frac{Rc_i}{2} \frac{V_{ref}}{V_{outifi}}$$

Dans le cas d'une tension $Vm_i$ continue de valeur $V_{ref}$, seul le traitement mis en oeuvre par l'unité de détection diffère de ce qui vient d'être décrit. Ce traitement peut être un simple filtrage passe-bas permettant d'extraire directement la composante continue $V_{outic}$ de la tension $V_{outi}$. Le dispositif de traitement 121 peut alors déterminer la valeur de la résistance série $Rs_{BRi}$ par la formule suivante :

$$Rs_{BRi} = Rc_i \frac{V_{ref}}{V_{outic}}$$

B) mesure de l'inductance propre $L_{BRi}$

Pour la mesure de l'inductance $L_{BRi}$ de la bobine $BR_i$, le générateur 331 commande l'application, via la source de tension commandable $433_i$, d'une tension $Vm_i$ alternative sinusoïdale sur la borne de la bobine $BR_i$ connectée à la source de tension $433_i$.

La tension $Vm_i$ peut, comme dans le cas A), s'exprimer comme suit :

$$Vm_i(t) = V_{ref} \cos(2\pi f_i t)$$

Cette fois ci, la fréquence $f_i$ de la tension $Vm_i$ est choisie proche ou supérieure à la fréquence de coupure de la bobine (égale à $RS_{BRi}/2\pi L_{BRi}$), de façon que l'impédance de l'inductance de la bobine $BR_i$ soit non négligeable devant la résistance série de la bobine $Rs_{BRi}$. A titre d'exemple, pour la mesure de l'inductance $L_{BRi}$, la fréquence $f_i$ est choisie comprise entre 1 et 50 fois la fréquence de coupure de la bobine. A titre d'exemple, pour la mesure de l'inductance $L_{BRi}$ la fréquence $f_i$ peut être comprise dans la plage allant de 1 à 20 kHz pour une fréquence de coupure de l'ordre de 1 kHz. De préférence, la fréquence $f_i$ est différente de la ou des fréquences d'émission du dispositif d'émission 101. A titre d'exemple, la fréquence $f_i$ est inférieure à la plus petite fréquence d'émission du dispositif d'émission 101, ou supérieure à la plus grande fréquence d'émission du dispositif d'émission 101. L'amplitude $V_{ref}$ de la tension $Vm_i$ est choisie de manière à ne pas saturer l'amplificateur opérationnel $203_i$.

La tension $Vm_i$ est amplifiée par l'étage d'amplification transimpédance $201_i$. L'image amplifiée de la tension $Vm_i$ forme ainsi une composante de fréquence $f_i$ de la tension de sortie $V_{outi}$ de l'étage d'amplification $201_i$, s'exprimant comme suit :

$$V_{outifi}(t) = V_{ref} \cos(2\pi f_i t) \frac{Rc_i}{Rs_{BRi} + L_{BRi} j\omega}$$

L'unité de détection du dispositif de traitement 121 est adaptée à extraire la valeur crête $V_{outifi}$ de la composante de fréquence $f_i$ du signal $V_{outi}$, s'exprimant comme suit :

$$V_{outifi} = \frac{V_{ref}}{2} \frac{Rc_i}{\sqrt{Rs_{BRi}^2 + L_{BRi}^2 \omega^2}}$$

A partir de cette valeur crête, le dispositif de traitement 121 peut déterminer la valeur de l'inductance $L_{BRi}$ par la formule suivante :

$$L_{BRi} = \frac{1}{\omega}\sqrt{\left(\frac{Rc_i}{2}\frac{V_{ref}}{V_{outifi}}\right)^2 - Rs_{BRi}^{\,2}}$$

C) mesure de l'inductance mutuelle $MI_{i,i'}$

Pour la mesure du coefficient d'inductance mutuelle $MI_{i,i'}$ entre deux bobines de réception $BR_i$ et $BR_{i'}$, le générateur 331 commande l'application, via la source de tension commandable $433_i$, d'une tension $Vm_i$ alternative sinusoïdale sur la borne de la bobine $BR_i$ connectée à la source de tension $433_i$. La tension $Vm_i$ peut, comme dans les cas A) et B), s'exprimer comme suit :

$$Vm_i(t) = V_{ref}\cos(2\pi f_i t)$$

Comme dans le cas B), la fréquence $f_i$ de la tension $Vm_i$ est choisie proche ou supérieure à la fréquence de coupure de la bobine $RS_{BRi}/2\pi L_{BRi}$, de façon que l'impédance de l'inductance de la bobine $BR_i$ soit non négligeable devant la résistance série $Rs_{BRi}$. A titre d'exemple, pour la mesure du coefficient d'inductance mutuelle, la fréquence $f_i$ est choisie comprise entre 1 et 50 fois la fréquence de coupure de la bobine. A titre d'exemple, pour la mesure du coefficient d'inductance mutuelle $MI_{i,i'}$, la fréquence $f_i$ est comprise dans la plage allant de 1 à 20 kHz pour une fréquence de coupure de la bobine de l'ordre de 1 kHz. De plus, comme dans le cas B), la fréquence $f_i$ est de préférence différente de la ou des fréquences d'émission du dispositif d'émission 101. A titre d'exemple, la fréquence $f_i$ est inférieure à la plus petite fréquence d'émission du dispositif d'émission 101, ou supérieure à la plus grande fréquence d'émission du dispositif d'émission 101. L'amplitude $V_{ref}$ de la tension $Vm_i$ est choisie de manière à ne pas saturer l'amplificateur opérationnel $203_i$.
La tension appliquée, via la source de tension commandable $433_{i'}$, sur la borne de la bobine $BR_{i'}$ connectée à la source de tension $433_{i'}$, est par exemple nulle. En tout état de cause, cette tension ne comporte pas de composante de fréquence $f_i$.
Le courant $i_{BRi}$ traversant la bobine $BR_i$ est relié à la tension de sortie $V_{outi}$ de l'amplificateur transimpédance $201_i$ par la relation suivante :

$$i_{BRi}(t) = \frac{V_{outi}(t)}{Rc_i}$$

Sous l'effet de ce courant sinusoïdal, la bobine $BR_i$ émet un champ électromagnétique alternatif de fréquence $f_i$. Ce champ est capté par la bobine $BR_{i'}$, induisant aux bornes de la bobine $BR_{i'}$ une tension $v_{BRi'}$ s'exprimant comme suit :

$$v_{BRi'}(t) = -j\omega MI_{i,i'}\,i_{BRi}(t)$$

Cette tension est amplifiée par l'étage d'amplification transimpédance $201_{i'}$, qui fournit à sa sortie une tension $V_{outi'}$ s'exprimant comme suit :

$$V_{outi'}(t) = -j\omega MI_{i,i'}\,i_{BRi}\cos(2\pi f_i t)\frac{Rc_{i'}}{Rs_{BRi'} + L_{BRi'}j\omega}$$

Le dispositif de traitement 121 est adapté, à partir des signaux mesurés en sortie des étages d'amplification $201_i$ et $201_{i'}$, à déterminer le coefficient $MI_{i,i'}$ qui s'exprime comme suit :

$$MI_{i,i'} = \frac{V_{outi'}}{V_{outi}}\frac{Rc_i}{Rc_{i'}}\frac{|Rs_{BRi} + L_{BRi}j\omega|}{\omega}$$

A titre d'exemple, les différents coefficients d'inductance mutuelle de l'ensemble 113 de bobines de réception, à savoir les coefficients $MI_{1,2}$, $MI_{1,3}$ et $MI_{1,3}$ dans l'exemple à trois bobines de réception, peuvent être déterminés simultanément en utilisant des fréquences d'excitation $f_i$ distinctes. A titre de variante, les différents coefficients d'inductance mutuelle peuvent être déterminés séquentiellement en utilisant une même fréquence d'excitation $f_i$.

Les mesures des paramètres $Rs_{BRi}$, $L_{BRi}$ et/ou $MI_{i,i'}$ peuvent être réalisées lors d'une phase de calibration préalable à une phase de suivi de position, et/ou pendant la phase de suivi de position proprement dite. Par exemple, les mesures des paramètres $Rs_{BRi}$, $L_{BRi}$ et/ou $MI_{i,i'}$ peuvent être réalisées en continu parallèlement aux mesures des champs électromagnétiques en provenance du dispositif d'émission, à condition que la ou les fréquences d'excitation appliquées par le système de mesure de paramètres sur les bobines de réception soient différentes de la ou les fréquences d'émission du dispositif d'émission. En outre, les paramètres $Rs_{BRi}$, $L_{BRi}$ et/ou $MI_{i,i'}$ peuvent être mesurés simultanément dès lors que les fréquences d'excitations utilisées pour les mesurer sont différentes les unes des autres.

A titre d'exemple, le générateur 331 peut comporter un synthétiseur numérique de fréquence (non représenté), adapté à générer un ou plusieurs signaux sinusoïdaux de fréquences commandables, et, le cas échéant, à additionner ces signaux. La source de tension commandable $433_i$ associée à chaque bobine comprend par exemple un convertisseur numérique-analogique (non représenté) dont l'entrée est reliée à une sortie numérique du synthétiseur de fréquences, et dont la sortie est reliée à la bobine $BR_i$. A titre de variante, un pont diviseur de tension résistif peut être prévu entre la sortie du convertisseur numérique-analogique et l'extrémité de la bobine $BR_i$ connectée à la source de tension $433_i$, pour ajuster le niveau de la tension d'excitation appliqué sur la bobine $BR_i$.

Un avantage du système de localisation décrit en relation avec les figures 3 et 4 est qu'il permet de mesurer d'éventuelles dérives de certains paramètres du dispositif de réception. Connaissant ces dérives, il est en particulier possible de corriger les valeurs de champ mesurées par le dispositif de réception, et ainsi d'améliorer la précision de localisation de l'ensemble de bobines de réception par rapport aux systèmes de localisation connus.

Des modes de réalisation particuliers ont été décrits. Diverses variantes et modifications apparaîtront à l'homme de l'art.

En particulier, les modes de réalisation décrits ne se limitent pas à l'application susmentionnée à la localisation d'outils chirurgicaux, mais peuvent s'appliquer à d'autres domaines nécessitant la localisation précise d'un objet dans un référentiel.

De plus, les modes de réalisation décrits ne sont pas limités à l'exemple susmentionné d'un système de localisation à trois bobines d'émission et trois bobines de réception, mais s'appliquent plus généralement à tout système électromagnétique de localisation comportant au moins une bobine d'émission et au moins une bobine de réception. On notera que dans le cas d'un système à une seule bobine de réception, il n'y a pas lieu de réaliser une mesure de coefficient d'inductance mutuelle.

Plus généralement, on notera que bien que l'on ait décrit un système permettant de mesurer d'éventuelles dérives des paramètres $Rs_{BRi}$, $L_{BRi}$ et/ou $MI_{i,i'}$, on pourra choisir, selon les besoins de l'application, de ne surveiller qu'un seul de ces paramètres, par exemple le paramètre $Rs_{BRi}$ qui est généralement le plus critique, ou un sous-ensemble de ces paramètres.

En outre, bien que l'on ait décrit ci-dessus un exemple de réalisation dans lequel les opérations réalisées par le dispositif de traitement 121 sont mises en oeuvre dans le domaine numérique, les modes de réalisation décrits ne se limitent pas à ce cas particulier. A titre de variante, les opérations réalisées par le dispositif 121 peuvent être mises en oeuvre en tout ou partie dans le domaine analogique. A titre d'exemple, les opérations de démodulation synchrone et de filtrage et/ou de détection synchrone peuvent être mises en oeuvre dans le domaine analogique, et les opérations de calcul de position à partir des valeurs de champ mesurées peuvent être mises en oeuvre dans le domaine numérique.

## Revendications

**1.** Système électromagnétique de suivi de position, comprenant :

un dispositif (101) d'émission d'un champ électro-magnétique comportant au moins une bobine d'émission ($BE_1$, $BE_2$, $BE_3$) et, connecté à ladite au moins une bobine d'émission, un premier générateur (101) d'un signal électrique d'excitation de ladite au moins une bobine d'émission ;
un dispositif de réception (111) du champ électro-magnétique émis par le dispositif d'émission (101), comportant au moins une bobine de réception ($BR_1$, $BR_2$, $BR_3$) et, connecté à ladite au moins une bobine de réception, un circuit de lecture (115) d'un signal électrique induit dans ladite au moins une bobine de réception ; et
un système de mesure d'au moins un paramètre du dispositif de réception (111), comprenant un deuxième générateur (331) d'un signal électrique d'excitation de ladite au moins une bobine de réception
**caractérisé en ce que**
ledit deuxième générateur (331) est directement connecté à ladite au moins une bobine de réception par une piste ou un fil conducteur.

**2.** Système selon la revendication 1, dans lequel ledit au moins un paramètre est un paramètre du groupe comprenant la résistance série ($Rs_{BRi}$) et l'inductance propre ($L_{BRi}$) de ladite au moins une bobine de réception ($BR_i$).

**3.** Système selon la revendication 1, dans lequel le dispositif de réception (111) comprend au moins deux bobines de réception ($BR_i$, $BR_{i'}$), et dans lequel ledit au moins un paramètre est un paramètre du groupe comprenant la résistance série ($Rs_{BRi}$) de chaque bobine de réception ($BR_i$), l'inductance propre ($L_{BRi}$) de chaque bobine de réception ($BR_i$), et le coefficient d'inductance mutuelle ($MI_{i,i'}$) entre deux bobines de réception ($BR_i$, $BR_{i'}$).

**4.** Système selon la revendication 2 ou 3, dans lequel ledit au moins un paramètre comprend la résistance série ($Rs_{BRi}$) de chaque bobine de réception ($BR_i$), et dans lequel le système de mesure est adapté, pour mesurer ladite résistance série, à appliquer sur une extrémité de la bobine de réception ($BR_i$) une tension continue ou une tension sinusoïdale de fréquence au moins 20 fois plus faible que la fréquence de coupure de la bobine.

**5.** Système selon la revendication 2 ou 3, dans lequel ledit au moins un paramètre comprend l'inductance propre ($L_{BRi}$) de chaque bobine de réception ($BR_i$), et dans lequel le système de mesure est adapté, pour mesurer ladite inductance propre, à appliquer sur une extrémité de la bobine de réception ($BR_i$) une tension sinusoïdale de fréquence supérieure à la fréquence de coupure de la bobine.

**6.** Système selon la revendication 3, dans lequel ledit au moins un paramètre comprend le coefficient d'inductance mutuelle ($MI_{i,i'}$) entre deux bobines de réception ($BR_i$, $BR_{i'}$), et dans lequel le système de mesure est adapté, pour mesurer ledit coefficient, à appliquer sur une extrémité de l'une des deux bobines de réception ($BR_i$) une tension sinusoïdale de fréquence supérieure à la fréquence de coupure de la bobine.

**7.** Système selon l'une quelconque des revendications 1 à 6, dans lequel le circuit de lecture (115) comprend, associé à chaque bobine de réception ($BR_i$), un étage d'amplification de lecture ($201_i$) comportant un amplificateur opérationnel ($203_i$) dont l'entrée inverseuse (-) est reliée à la sortie par l'intermédiaire d'une résistance de contre-réaction ($Rc_i$) et dont l'entrée non inverseuse (+) est reliée à un noeud (GND) d'application d'un potentiel de référence, une première extrémité de la bobine de réception ($BR_i$) étant connectée à l'entrée inverseuse (-) de l'amplificateur opérationnel.

**8.** Système selon la revendication 7, dans lequel le deuxième générateur (331) comprend, associé à chaque bobine de réception ($BR_i$), une source de tension commandable ($433_i$) connectée entre la deuxième extrémité de la bobine de réception ($BR_i$) et ledit noeud (GND) d'application d'un potentiel de référence.

**9.** Système selon l'une quelconque des revendications 1 à 8, dans lequel le deuxième générateur (331) comprend un synthétiseur numérique de fréquences.

**10.** Système selon l'une quelconque des revendications 1 à 9, dans lequel ladite au moins une bobine d'émission comprend trois bobines d'émission (BE1, BE2, BE3) orientées selon des axes distincts, et dans lequel ladite au moins une bobine de réception comprend trois bobines de réception (BR1, BR2, BR3) orientées selon des axes distincts.

**Patentansprüche**

**1.** Ein elektromagnetisches Positionsverfolgungssystem, das Folgendes aufweist:

eine elektromagnetische Feldemissionsvorrichtung (101), die wenigstens eine Emitterspule ($BE_1$, $BE_2$, $BE_3$) aufweist und verbunden mit der wenigstens einen Emitterspule einen ersten Generator (101) eines elektrischen Signals zum Erregen der wenigstens einen Emitterspule;
eine Vorrichtung (111) zum Empfangen des von der Emissionsvorrichtung (101) emittierten elektromagnetischen Feldes, die wenigstens eine Empfangsspule ($BR_1$, $BR_2$, $BR_3$) aufweist, und verbunden mit der wenigstens einen Empfangsspule eine Schaltung (115) zum Lesen eines elektrischen Signals, das in der wenigstens einen Empfangsspule induziert wird; und
ein System zum Messen wenigstens eines Parameters der Empfangsvorrichtung (111), das einen zweiten Generator (331) eines elektrischen Signals aufweist zum Erregen der wenigstens einen Empfangsspule, **dadurch gekennzeichnet, dass**
der zweite Generator (331) direkt mit der wenigstens einen Empfangsspule durch eine Leiterbahn oder einen Leiterdraht verbunden ist.

2. System nach Anspruch 1, wobei der wenigstens eine Parameter ein Parameter aus der Gruppe ist, die den Serienwiderstand ($RS_{BRi}$) und die Eigeninduktivität ($L_{BRi}$) der wenigstens einen Empfangsspule ($BR_i$) aufweist.

3. System nach Anspruch 1, wobei die Empfangsvorrichtung (111) wenigstens zwei Empfangsspulen ($BR_i$, $BR_{i'}$) aufweist und wobei der wenigstens eine Parameter ein Parameter aus der Gruppe ist, die den Serienwiderstand ($RS_{BRi}$) von jeder Empfangsspule ($BR_i$), die Eigeninduktivität ($L_{BRi}$) von jeder Empfangsspule ($BR_i$) und den Koeffizienten ($MI_{i,i'}$) der gemeinsamen Induktivität zwischen zwei Empfangsspulen ($BR_i$, $BR_{i'}$) aufweist.

4. System nach Anspruch 2 oder 3, wobei der wenigstens eine Parameter den Serienwiderstand ($RS_{BRi}$) von jeder Empfangsspule ($BR_i$) aufweist und wobei das Messsystem geeignet ist, den Serienwiderstand zu messen und an ein Ende der Empfangsspule ($BR_i$) eine Gleichspannung oder eine Sinusspannung mit einer Frequenz anzulegen, die wenigstens 20 mal geringer als die Grenzfrequenz der Spule ist.

5. System nach Anspruch 2 oder 3, wobei der wenigstens eine Parameter die Eigeninduktivität ($L_{BRi}$) von jeder Empfangsspule ($BR_i$) aufweist und wobei das Messsystem geeignet ist, die Eigeninduktivität zu messen und an ein Ende der Empfangsspule ($BR_i$) eine Sinusspannung mit einer Frequenz größer als die Grenzfrequenz der Spule anzulegen.

6. System nach Anspruch 3, wobei der wenigstens eine Parameter den Koeffizienten der gemeinsamen Induktivität ($MI_{i,i'}$) zwischen zwei Empfangsspulen ($BR_i$, $BR_{i'}$) aufweist und wobei das Messsystem geeignet ist, den Koeffizienten zu messen und an ein Ende von einer der zwei Empfangsspulen ($BR_i$) eine Sinusspannung mit einer Frequenz größer als die Grenzfrequenz der Spule anzulegen.

7. System nach einem der Ansprüche 1 bis 6, wobei die Leseschaltung (115) assoziiert mit jeder Empfangsspule ($BR_i$) eine Abfühlverstärkerstufe ($201_i$) aufweist, die einen Funktionsverstärker ($203_i$) aufweist, dessen invertierter Eingang (-) an den Ausgang durch einen Rückkopplungswiderstand ($Rc_i$) gekoppelt ist und dessen nichtinvertierter Eingang (+) an einen Knoten (GND) des Anlegens eines Referenzpotenzials gekoppelt ist, wobei ein erstes Ende der Empfangsspule ($BR_i$) mit dem invertierten Eingang (-) des Funktionsverstärkers verbunden ist.

8. System nach Anspruch 7, wobei der zweite Generator (331) assoziiert mit jeder Empfangsspule ($BR_i$) eine steuerbare Spannungsquelle ($433_i$) aufweist, die zwischen dem zweiten Ende der Empfangsspule ($BR_i$) und dem Knoten (GND) des Anlegens eines Referenzpotentials verbunden ist.

9. System nach einem der Ansprüche 1 bis 8, wobei der zweite Generator (331) einen digitalen Frequenzsynthesizer aufweist.

10. System nach einem der Ansprüche 1 bis 9, wobei die wenigstens eine Emitterspule drei Emitterspulen (BE1, BE2, BE3) aufweist, die entlang verschiedener Achsen angeordnet sind und wobei die wenigstens eine Empfangsspule drei Empfangsspulen (BR1, BR2, BR3) aufweist, die entlang verschiedener Achsen angeordnet sind.

**Claims**

1. An electromagnetic position tracking system comprising:

an electromagnetic field emission device (101) comprising at least one emitter coil ($BE_1$, $BE_2$, $BE_3$) and, connected to said at least one emitter coil, a first generator (101) of an electric signal for exciting said at least one emitter coil;

a device (111) for receiving the electromagnetic field emitted by the emission device (101), comprising at least one receiver coil ($BR_1$, $BR_2$, $BR_3$) and, connected to said at least one receiver coil, a circuit (115) for reading an electric signal induced in said at least one receiver coil; and

a system for measuring at least one parameter of the reception device (111), comprising a second generator (331) of an electric signal for exciting said at least one receiver coil **characterized in that**

said second generator (331) is directly connected to said at least one receiver coil by a conductive track or wire.

2. The system of claim 1, wherein said at least one parameter is a parameter from the group comprising the series resistance ($Rs_{BRi}$) and the self-inductance ($L_{BRi}$) of said at least one receiver coil ($BR_i$).

3. The system of claim 1, wherein the reception device (111) comprises at least two receiver coils ($BR_i$, $BR_{i'}$), and wherein said at least one parameter is a parameter from the group comprising the series resistance ($Rs_{BRi}$) of each receiver coil ($BR_i$), the self-inductance ($L_{BRi}$) of each receiver coil ($BR_i$), and the coefficient ($MI_{i,i'}$) of mutual inductance between two receiver coils ($BR_i$, $BR_{i'}$).

4. The system of claim 2 or 3, wherein said at least one parameter comprises the series resistance ($Rs_{BRi}$) of each receiver coil ($BR_i$), and wherein the measurement system is capable, to measure said series resistance, of applying on one end of the receiver coil ($BR_i$) a DC voltage or a sinusoidal voltage having a frequency at least 20 times lower than the cut-off frequency of the coil.

5. The system of claim 2 or 3, wherein said at least one parameter comprises the self-inductance ($L_{BRi}$) of each receiver coil ($BR_i$), and wherein the measurement system is capable, to measure said self-inductance, of applying on one end of the receiver coil ($BR_i$) a sinusoidal voltage having a frequency greater than the cut-off frequency of the coil.

6. The system of claim 3, wherein said at least one parameter comprises the coefficient of mutual inductance ($MI_{i,i'}$) between two receiver coils ($BR_i$, $BR_{i'}$), and wherein the measurement system is capable, to measure said coefficient, of applying on one end of one of the two receiver coils ($BR_i$) a sinusoidal voltage having a frequency greater than the cut-off frequency of the coil.

7. The system of any of claims 1 to 6, wherein the read circuit (115) comprises, associated with each receiver coil ($BR_i$), a sense amplification stage ($201_i$) comprising an operational amplifier ($203_i$) having its inverting input (-) coupled to the output via a feedback resistor ($Rc_i$) and having its non-inverting input (+) coupled to a node (GND) of application of a reference potential, a first end of the receiver coil ($BR_i$) being connected to the inverting input (-) of the operational amplifier.

8. The system of claim 7, wherein the second generator (331) comprises, associated with each receiver coil ($BR_i$), a controllable voltage source ($433_i$) connected between the second end of the receiver coil ($BR_i$) and said node (GND) of application of a reference potential.

9. The system of any of claims 1 to 8, wherein the second generator (331) comprises a digital frequency synthesizer.

10. The system of any of claims 1 to 9, wherein said at least one emitter coil comprises three emitter coils (BE1, BE2, BE3) oriented along different axes, and wherein said at least one receiver coil comprises three receiver coils (BR1, BR2, BR3) oriented along different axes.

Fig 1

Fig 2

Fig 3

Fig 4

**EP 3 120 798 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050165297 A